# EUROPÄISCHE PATENTANMELDUNG

(11) **EP 1 184 358 A2**
(43) Veröffentlichungstag der Anmeldung: **06.03.2002**
(21) Anmeldenummer: 01117668.2
(22) Anmeldetag: 19.07.2001
(51) Int. Cl.: C07B 31/00, C07B 63/00

(54) **Verfahren zur Aufarbeitung und Reinigung der Reaktionsprodukte aus der Reduktion mit Lithiumaluminiumhydrid**

(30) Priorität: 01.08.2000 DE 10037408
(71) Anmelder: BAYER AG, 51368 Leverkusen (DE)
(72) Erfinder: Scherer, Johannes, Dr., 51375 Leverkusen (DE); Rauchschwalbe, Günter, Dr., 51375 Leverkusen (DE); Hammerschmidt, Erich, Dr., 51467 Bergisch Gladbach (DE); Hartges, Heinz-Gerd, 51381 Leverkusen (DE)

(57) **Zusammenfassung**

Basische reagierende, organische, stickstoffhaltige Verbindungen werden durch Reduktion mit Lithiumaluminiumhydrid in besonders vorteilhafter Weise erhalten, wenn man das nach der Reduktion vorliegende Gemisch hydrolysiert, indem man es zur einer wässrigen Lösung hinzufügt, in der Substanzen gelöst sind, die α-Hydroxycarboxyl- und/oder a-Aminocarboxyl-Gruppen enthalten und die sich dabei bildenden beiden Phasen bei einem pH-Wert der wässrigen Phase von 7 bis 12 trennt.

## Beschreibung

Die vorliegende Erfindung betrifft ein Verfahren zur Herstellung basisch reagierender, organischer, stickstoffhaltiger Verbindungen durch Reduktion mit Lithiumaluminiumhydrid und anschließender Zersetzung des Reaktionsgemisches unter Bildung eines zweiphasigen, wässrig-organischen Systems.

Bei den basisch reagierenden, organischen, stickstoffhaltigen Verbindungen kann es sich beispielsweise um Amine, Pyridine, Piperidine, Piperazine, Pyrrole, Pyrrolidine oder Indole handeln.

Basisch reagierende, organische, stickstoffhaltige Verbindungen sind wichtige Zwischenprodukte für die Herstellung von Pflanzenschutzmitteln und pharmazeutischen Wirkstoffen. So sind z.B. N-Benzyl-pyrrolopiperidin und daraus herstellbare Verbindungen wie Pyrrolopiperidin als Komponenten antibiotisch wirksamer pharmazeutischer Verbindungen von Interesse (s. z.B. DE-A 4 234 330).

Amine, Pyridine, Piperidine, Piperazine, Pyrrole, Pyrrolidine und Indole kann man in bekannter Weise z.B. durch Reduktion von aminogruppen-, pyridinogruppen-, pyrrologruppen- oder indolgruppenhaltigen Verbindungen, z.B. durch Reduktion von Aminosäuren, Aminoketonen, Pyridincarbonsäuren, Indolylcarbonsäuren, Pyrrolidinonen, Pyrrolidindionen, Piperidinonen, Piperidindionen, Piperazinonen oder Piperazindionen mit Lithiumaluminiumhydrid herstellen, Amine z.B. auch durch Reduktion entsprechender Amide, Imide, Imine, Hydrazine, Hydrazone, Hydroxylamine, Oxime und Nitrile mit Lithiumaluminiumhydrid.

Bei derartigen Reduktionen mit Lithiumaluminiumhydrid entstehen Addukte, die zur Produktisolierung zersetzt werden müssen. Außerdem wird Lithiumaluminiumhydrid meist im Überschuß eingesetzt, was zur Folge hat, dass auch der Lithiumaluminium-hydrid-Überschuß vor der Produktisolierung zersetzt werden muß. Diese Zersetzungen erfolgen nach dem Stand der Technik durch Zugabe von wässriger Natronlauge, wasserhaltigen organischen Lösungsmitteln und/oder Wasser, wobei sich stets voluminöse Niederschläge bilden, die nur schwierig abzutrennen sind und an denen die hergestellten basisch reagierenden, organischen, stickstoffhaltigen Verbindungen adsorbiert werden.

Eine Aufarbeitung des nach der Reaktion mit Lithiumaluminiumhydrid vorliegenden Gemisches durch Zugabe von Säuren verhindert zwar die Bildung von Aluminiumhydroxid- und/oder Aluminat-Niederschlägen. Sie ist jedoch bei der Herstellung von basisch reagierenden, organischen, stickstoffhaltigen Verbindungen nicht anwendbar, weil sonst diese Verbindungen Salze bilden würden, die in der wässrigen Phase löslich und daraus durch Phasentrennung oder Extraktion nicht mehr abtrennbar sind.

Man kann auch so verfahren, dass man genau soviel Wasser zugibt, wie theoretisch benötigt wird, um das Aluminiumaddukt und überschüssiges Lithiumaluminiumhydrid zu zersetzen. Es bildet sich dann ein flaumiger Aluminium- und Lithiumhydroxid-Niederschlag (Lithium Aluminium Hydride Industrial Use, Firmenschrift Chemetall, Frankfurt 1993). Die Dosierung einer genauen Wassermenge ist unter den gegebenen Bedingungen jedoch schwierig.

Schließlich ist vorgeschlagen worden, bei der Aufarbeitung von Reaktionsgemischen, die bei Reduktionen mit Lithiumaluminiumhydrid anfallen, zunächst Wasser zuzusetzen und dann den ausgefallenden Niederschlag durch Zugabe einer 20 gew.-%igen wässrigen Lösung von Kalium-Natrium-Tartrat im Überschuß wieder aufzulösen (s. J.A.C.S. 70, 3788 (1948)). Auch dieses Verfahren weist eine Reihe von Nachteilen auf:
- Die Zugabe von Wasser ins Reaktionsgemisch führt zu einer heftigen Reaktion, die allenfalls im Labormaßstab, nicht jedoch im halbtechnischem oder technischem Maßstab beherrschbar ist.
- Es wird zunächst auch hier ein Niederschlag gebildet, der die Rührbarkeit des Gemisches negativ beeinflußt. Erst anschließend wird er wieder aufgelöst.
- Es wird mit geringen Reagenz-Konzentrationen gearbeitet (7,6 g Lithiumaluminiumhydrid in 500 ml Lösungsmittel und 500 ml 20 gew.-%ige Kalium-Natrium-Tartrat-Lösung), was die Handhabung großer Volumina bedeutet und mit geringen Raum-Zeit-Ausbeuten einhergeht.
- Das Kalium-Natrium-Tartrat ist in großem Überschuß einzusetzen (ca. 2,4 Mol = 4,8 Äquivalente pro Mol Lithiumaluminiumhydrid).

Es besteht also immer noch ein Bedürfnis nach einem Verfahren zur Herstellung von basisch reagierenden, organischen, stickstoffhaltigen Verbindungen durch Reduktion mit Lithiumaluminiumhydrid, bei dem diese Nachteile nicht auftreten.

Es wurde nun ein Verfahren zur Herstellung von basisch reagierenden, organischen, stickstoffhaltigen Verbindungen durch Reduktion mit Lithiumaluminiumhydrid gefunden, das dadurch gekennzeichnet ist, dass man das nach der Reduktion vorliegende Gemisch hydrolysiert, indem man es zu einer wässrigen Lösung hinzufügt, in der Substanzen gelöst sind, die α-Hydroxycarboxyl- und/oder α-Aminocarboxyl-Gruppen enthalten und die sich dabei bildenden beiden Phasen bei einem pH-Wert der wässrigen Phase von 7 bis 12 trennt.

Das erfindungsgemäße Verfahren kann man z.B. anwenden auf die Herstellung von aliphatischen und aromatischen Aminen, Pyridinen, Piperidinen, Piperazinen, Pyrrolen, Pyrrolidinen und Indolen. Diese kann man beispielsweise durch Reduktion von Verbindungen erhalten, die neben Amino-, Pyridino-, Piperidino-, Piperazino-, Pyrrolo-, Pyrrolidino- und/oder Indolgruppen eine oder mehrere, mit Lithiumaluminiumhydrid reduzierbare Gruppen, z.B. Sauerstoff enthaltende Gruppen wie Carboxylat- und/oder Ketogruppen, enthalten. Amine kann man z.B. auch durch Reduktion der entsprechenden Amide, Imide, Imine, Hydrazone, Hydroxylamine, Oxime oder Nitrile erhalten.

Erfindungsgemäß herstellbare Amine sind z.B. solche der Formel 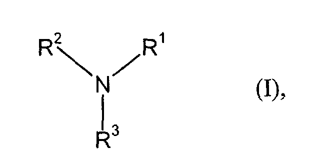 in der
- R¹, R² und R³: unabhängig voneinander jeweils für C₁-C₈-Alkyl, Phenyl, Benzyl oder (CH)ₙX stehen,
wobei n für eine ganze Zahl von 1 bis 4 und X für OR⁷ oder NR⁷R⁸ stehen mit R⁷ und R⁸ = Wasserstoff, C₁-C₈-Alkyl, Phenyl oder Benzyl oder
- R¹ und R²: unabhängig voneinander für C₁-C₆-Alkyl und R³ für C₄-C₂₀-Alkyl, Phenyl, Benzyl oder Ethyl stehen.

Erfindungsgemäß herstellbare Pyridine, Piperidine und Piperazine sind z.B. solche der Formeln 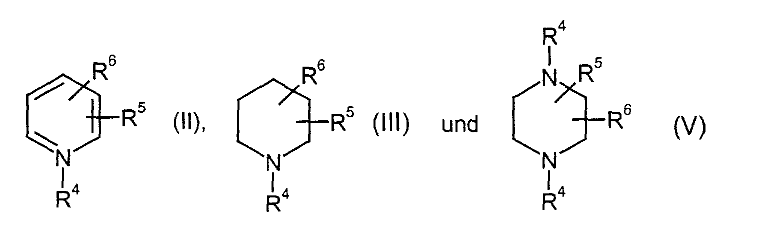 in denen
- R⁴, R⁵ und R⁶: unabhängig voneinander für Wasserstoff, C₁-C₈-Alkyl, Phenyl, Benzyl, X oder (CH)ₙX stehen,
wobei n für eine ganze Zahl von 1 bis 4 und X für OR⁷ oder NR⁷R⁸ stehen mit R⁷ und R⁸ = Wasserstoff, C₁-C₈-Alkyl, Phenyl oder Benzyl
oder
R⁴ die vorstehende Bedeutung hat und R⁵ und R⁶ gemeinsam, eine -CH₂-NR⁹-CH₂-Brücke bilden, bei der R⁹ für Wasserstoff, C₁-C₈-Alkyl, Phenyl oder Benzyl steht.

Erfindungsgemäß herstellbare Indole sind z.B. solche der Formel 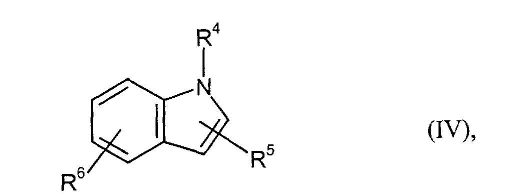 in der
R⁴, R⁵ und R⁶ die bei den Formeln (II), (III) und (V) angegebenen Bedeutungen haben, R⁵ und R⁶ jedoch nicht gemeinsam für eine -CH₂-NR⁹-CH₂-Brücke stehen können.

Erfindungsgemäß herstellbare Pyrrole und Pyrrolidine sind z.B. solche der Formeln (VI) und (VII) 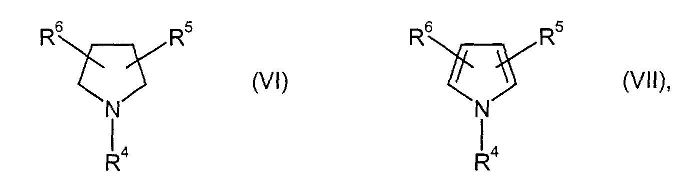 in denen
R⁴, R⁵ und R⁶ die bei den Formeln (II), (III) und (V) angegebene Bedeutung haben.

Vorzugsweise werden Verbindungen der Formeln (II), (III), (V)und (VI) hergestellt, bei denen
- R⁴: für Wasserstoff, Methyl, Ethyl, Phenyl, Benzyl oder (CH₂)ₙX steht und
- R⁵ und R⁶: unabhängig voneinander für Wasserstoff, C₁-C₄-Alkyl, Phenyl, Benzyl, X oder (CH)ₙX stehen,
wobei n für 1 oder 2 und X für OR⁷ oder NR⁷R⁸ stehen mit R⁷ und R⁸ Wasserstoff, Methyl, Ethyl, Phenyl oder Benzyl oder
- R⁴: die vorstehende Bedeutung hat und R⁵ und R⁶ gemeinsam eine -CH₂-NR⁹-CH₂-Brücke bilden, bei der R⁹ für Wasserstoff, Methyl, Ethyl, Phenyl oder Benzyl steht.

Besonders bevorzugt werden Verbindungen der Formeln (II), (III) und (VI) hergestellt, bei denen
- R⁴: für Wasserstoff, Methyl, Ethyl, Phenyl oder Benzyl steht und
- R⁵ und R⁶: unabhängig voneinander für Wasserstoff, C₁-C₄-Alkyl, Phenyl, Benzyl, X oder (CH)ₙX stehen,
wobei n für 1 oder 2 und X für OH oder NH₂ stehen, oder
- R⁴: die vorstehende Bedeutung hat und R⁵ und R⁶ gemeinsam eine -CH₂-NR⁹-CH₂-Brücke bilden, bei der R⁹ für Wasserstoff, Methyl, Ethyl, Phenyl oder Benzyl steht.

Besonders bevorzugt stellt man erfindungsgemäß 3-Amino-1-benzylpyrrolidin oder 8-Benzyl-2,8-diazabicyclo[4.3.0]-nonan her, das auch als N-Benzyl-pyrrolopiperidin oder kurz BEPP bezeichnet wird, wobei man 3-Amino-1-benzyl-pyrrolidindion bzw. 8-Benzyl-7,9-dioxo-2,8-diazabicyclo[4.3.0]-nonen, das auch kurz als DOPP bezeichnet wird, mit Lithiumalumiumhydrid reduziert.

Die Reduktion mit Lithiumaluminiumhydrid kann auf an sich bekannte Weise durchgeführt werden (s. z.B. Lithium Aluminium Hydride Industrial Use, Firmenschrift Chemetall, Frankfurt (1993)). Üblicherweise arbeitet man bei Temperaturen im Bereich 5 bis 60°C, in Gegenwart eines Lösungsmittels und mit 0,25 bis 4 mol Lithiumaluminiumhydrid pro Äquivalent zu reduzierender Substanz. Als Lösungsmittel sind beispielsweise acyclische und cyclische Mono- und Oligoether geeignet, die z.B. 4 bis 10 C-Atome enthalten, wie Diethylether, Diisopropylether, Methyl-tert.-butylether, Dibutylether, Ethylenglykoldimethylether, Diethylenglykoldimethylether, Tetraethylenglykoldimethylether, Tetrahydrofuran, 2-Methyl-tetrahydrofuran und Dioxan. Die Ether können auch als Gemisch untereinander und/oder im Gemisch mit anderen, gegenüber Lithiumaluminiumhydrid inerten organischen Lösungsmitteln, beispielsweise Kohlenwasserstoffen wie Benzol, Toluol, Xylol und/oder Petrolether, eingesetzt werden.

Es ist ein wesentliches Merkmal der vorliegenden Erfindung, dass man das nach Beendigung der Reduktion mit Lithiumaluminiumhydrid vorliegende Gemisch zu einer wässrigen Lösung hinzufügt, in der Substanzen gelöst sind, die α-Hydroxycarboxyl- und/oder α-Aminocarboxyl-Gruppen enthalten.

Als α-Hydroxycarboxylgruppen enthaltende Substanzen kommen beispielsweise α-Hydroxycarbonsäure mit 1 bis 6 OH-Gruppen und 2 bis 10 C-Atomen infrage, wobei sich mindestens eine OH-Gruppe in α-Stellung zu einer Carboxylgruppe befindet. Das Kohlenstoffgerüst solcher α-Hydroxycarbonsäuren kann geradkettig oder verzweigt sein. Bevorzugte α-Hydroxycarbonsäuren sind solche mit 1 bis 5 OH-Gruppen und 2 bis 6 C-Atomen wie Glykolsäure, Tartronsäure, Äpfelsäure, Zitronensäure, Weinsäure, Gluconsäure, Zuckersäuren, Mannozuckersäure und Schleimsäure.

Als α-Aminocarboxylgruppen enthaltende Substanzen kommen beispielsweise α-Aminocarbonsäuren mit 1 bis 4 Aminogruppen und 2 bis 20 C-Atomen infrage, wobei sich mindestens eine primäre, sekundäre oder tertiäre Aminogruppe in α-Stellung zu einer Carboxylgruppe befindet. Das Kohlenstoffgerüst solcher α-Amino-carbonsäuren kann geradkettig oder verzweigt sein. Bevorzugte α-Aminocarbonsäuren sind solche mit 1 bis 3 Aminogruppen und 2 bis 14 C-Atomen wie Glycin, Alanin, Asparaginsäure, Asparagin, Glutaminsäure, Glutamin, Nitrilotriessigsäure, Ethylendiamintetraessigsäure, Diethylentriaminpentaessigsäure und Iminodibernsteinsäure.

Es können auch wässrige Lösungen eingesetzt werden, in denen mehrere α-Hydroxycarboxylgruppen enthaltende Substanzen, mehrere α-Aminocarboxylgruppen enthaltende Substanzen oder gemeinsam α-Hydroxycarboxylgruppen enthaltende Substanzen und α-Aminocarboxylgruppen enthaltende Substanzen vorliegen.

Carboxylgruppen können in Form freier Carbonsäurereste (COOH) oder in Salzform vorliegen, beispielsweise als Alkalisalze (z.B. COO- Na⁺). Aminogruppen können in freier Form vorliegen (-NH₂, -NHR oder -NR₂) oder als Ammoniumsalze (z.B. -NH₃⁺ Cl-).

Im folgenden werden α-Hydroxycarboxylgruppen und α-Aminocarboxylgruppen enthaltende Substanzen gemeinsam auch als HAC bezeichnet. Ein Äquivalent HAC bedeutet diejenige Menge HAC, die ein Mol α-Hydroxylcarboxylgruppen und α-Aminocarboxylgruppen enthält.

Es ist vorteilhaft, nicht weniger als 1 Äquivalent HAC pro Mol eingesetztes Lithiumaluminiumhydrid zu verwenden und zu große Überschüsse an HAC zu vermeiden. Beispielsweise kann man die Bildung von unerwünschten Niederschlägen während der Aufarbeitung des Reaktionsgemisches aus der Reduktion mit Lithiumaluminiumhydrid vermeiden, wenn man 1 bis 3 Äquivalente HAC einsetzt. Vorzugsweise setzt man 1 bis 2 Äquivalente HAC zu.

Die HAC können in wässrigen Lösungen mit einer Konzentration von beispielsweise 5 bis 60 Gew.-%, vorzugsweise 10 bis 40 Gew.-% eingesetzt werden.

Die erfindungsgemäß durchzuführende Phasentrennung kann beispielsweise bei Temperaturen von 5 bis 100°C durchgeführt werden. Vorzugsweise arbeitet man 30 bis 80°C. Wenn die für die Phasentrennung gewählte Temperatur höher liegt als der Siedepunkt einer Komponente des zu trennenden Gemisches kann die Phasentrennung auch unter Druck durchgeführt werden.

Vor der Phasentrennung beträgt der pH-Wert der wässrigen Phase vorzugsweise 7,5 bis 9,5.

Falls erforderlich oder erwünscht kann nach der Hydrolyse der pH-Wert für die Phasentrennung durch Zugabe von alkalisch reagierenden anorganischen Substanzen wie Natriumhydroxid, Kaliumhydroxid, Calciumhydroxid, Soda oder Ammoniak auf den gewünschten pH-Wert eingestellt werden. Man kann alkalisch reagierende anorganische Substanzen als solche aber auch z.B. in Form von wässrigen Lösungen zufügen.

Weiterhin ist es vorteilhaft, das Gemisch vor der Phasentrennung intensiv zu rühren.

Die erfindungsgemäß hergestellte basisch reagierende, organische, stickstoffhaltige Verbindung befindet sich nach der Phasentrennung zum größten Teil in der oberen organischen Phase. Weitere Anteile der basisch reagierenden, organischen, stickstoffhaltigen Verbindung kann man erhalten, indem man nach der Phasentrennung die wässrige Phase extrahiert. Als Extraktionsmittel eignen z.B. die oben für die Durchführung der Reduktion beschriebenen Lösungsmittel und Lösungsmittelgemische oder andere, mit Wasser nicht oder nur wenig mischbare Lösungsmittel.

Weitere Anteile der basisch reagierenden, organischen, stickstoffhaltigen Verbindungen kann man in die organische Phase bringen, wenn man vor der Phasentrennung Elektrolyte zufügt, die einen Aussalzeffekt bewirken. Beispiele für Elektrolyte sind Salzsäure, Schwefelsäure, Phosphorsäure, Salpetersäure, Bromwasserstoffsäure und Alkali- und Ammoniumchloride, -bromide, -sulfate, -hydrogensulfate, -phosphate, -hydrogenphosphate, -dihydrogenphosphate und -nitrate.

Die hergestellte Verbindung liegt schließlich in Form einer Lösung in dem für die Reduktion eingesetztem Lösungsmittel vor, gegebenenfalls zusätzlich in Form eines organischen Extraktes aus der wässrigen Phase. Man kann die hergestellten Verbindungen häufig in dieser Form direkt weiter verwenden. Gewünschtenfalls kann man auch das Lösungs- bzw. Extraktionsmittel entfernen, z.B. durch Abdestillieren, gegebenenfalls unter vermindertem Druck.

Das erfindungsgemäße Verfahren hat eine Reihe von überraschenden Vorteilen:
- Durch (gegebenenfalls dosierte) Zugabe des nach der Reduktion vorliegenden Gemisches in eine wässrige Lösung, die Substanzen mit α-Hydroxycarboxyl-und/oder α-Aminocarboxyl-Gruppen enthält, kann die Zersetzung auch von großen Mengen des Gemisches unter Kontrolle der Wärme- und Wasserstoffentwicklung durchführt werden.
- Während des Verfahrens bilden sich keine Niederschläge, die abgetrennt oder aufgelöst werden müssen.
- Während der Zersetzung bilden sich keine Krusten und Klumpen, die unzersetztes Reduktionsgemisch einschließen können.
- Die Phasentrennung erfolgt ohne Mulm- und ohne Emulsionsbildung.
- Das Gefäß, in dem die Reduktion durchgeführt wurde, bleibt frei von Feuchtigkeit und muß deshalb vor dem nächsten Ansatz nicht gesondert in aufwendiger Weise getrocknet werden.
- Die Reduktion kann mit höheren Reagenz-Konzentrationen durchgeführt werden.
- Es kann mit weniger Substanz, die α-Hydroxycarboxyl- und/oder α-Aminocarboxyl-Gruppen enthält, gearbeitet werden als im andersartigen Verfahren nach J.A.C.S. 70, 3738 (1970).

### Beispiele

### Beispiel 1

In einem Kolben wurden unter Stickstoff in einem Gemisch aus 315 g trockenem Tetrahydrofuran und 135 g trockenem Toluol 52,9 g Lithiumaluminiumhydrid vorgelegt. Zur dieser Suspension wurden bei 90°C 169,4 g geschmolzenes DOPP aus einem auf 110°C beheizten Tropftrichter zudosiert. Die anfangs mäßige Wasserstoffentwicklung wurde mit der Zeit lebhafter. Die Rückflußtemperatur fiel kontinuierlich auf 77°C ab. Nach beendeter Zugabe wurde noch 5 Stunden unter Rückfluß nachgerührt und die Suspension dann auf 20°C abgekühlt. Die dünnflüssige Suspension wurde in einem trockenem, mit Stickstoff gespülten Tropftrichter überführt. In einem zweiten Kolben wurden 340,2 g Citronensäure (Monohydrat) in 1079,4 g 7,4 gew.-%ige Schwefelsäure vorgelegt. Zu dieser Lösung wurde aus dem Tropftrichter die Reaktionssuspension im Verlauf einer Stunde bei 10°C zudosiert (Wasserstoffentwicklung). Es bildete sich eine organische und eine wässrige Phase. Es wurde eine Stunde bei 60°C nachgerührt. Nach dem Abkühlen des Kolbeninhalts auf 30°C wurde der pH-Wert durch Zugabe von 990 g wässriger Natronlauge (15 gew.-%ig) auf den Wert 8,5 eingestellt. Danach wurde die Temperatur auf 50°C erhöht und bei dieser Temperatur eine Stunde gerührt. Die organische Phase wurde dann abgetrennt und die wässrige Phase mit einem Gemisch aus 280 g Tetrahydrofuran und 120 g Toluol bei 50°C extrahiert. Die abgetrennte organische Phase enthielt 120 g reines N-Benzyl-pyrrolo-piperidin (BEPP), das Extrakt 10 g BEPP. In der wässrigen Phase wurden noch 0,5 g BEPP analysiert (HPLC, externer Standard). Die beiden organischen Phasen wurden vereinigt und die Lösungsmittel zum größten Teil im Vakuum abgezogen. Es blieben 149,5 g eines Öls zurück, das 86,5 Gew.-% BEPP (HPLC, externer Standard) enthielt. Das entspricht einer Ausbeute von 88,1 % der Theorie.

### Beispiel 2

In einem Kolben wurden unter Stickstoff 370 g 2-Methyltetrahydrofuran und 76 g Lithiumaluminiumhydrid vorgelegt. Zu der Suspension wurde bei 103°C aus einem auf 40°C beheizten Tropftrichter eine Lösung von 244 g DOPP in 163 g 2-Methyl-tetrahydrofuran zugetropft. Die anfangs mäßige Wasserstoffentwicklung wurde mit der Zeit lebhafter, wobei die Rückflußtemperatur kontinuierlich auf 78°C fiel. Nach Beendigung der DOPP-Zugabe wurde noch 3 Stunden unter Rückfluß gerührt, dann auf 20°C abgekühlt. Die danach vorliegende Suspension wurde in einen trockenen, mit Stickstoff gespülten Tropftrichter überführt. In einem zweiten Kolben wurde 504 g Zitronensäure-monohydrat in 1017,6 g 11,6 gew.-%iger Schwefelsäure vorgelegt. Zu dieser Lösung wurde aus dem Tropftrichter die Suspension während einer Stunde bei 10°C zudosiert. Es wurde 1 Stunde bis 60°C nachgerührt. Nach dem Abkühlen des Kolbeninhalts auf 30°C wurde bei dieser Temperatur der pH-Wert durch Zugabe von 443 g 15 gew.-%ige wässriger Natronlauge auf einen Wert von 8,5 eingestellt. Danach wurde die Temperatur auf 60°C gesteigert und bei dieser Temperatur 1 Stunde nachgerührt. Die organische Phase wurde abgetrennt und die wässrige Phase mit 533 g 2-Methyltetrahydrofuran extrahiert. Die organische Phase enthielt 180 g reines BEPP, das Extrakt 10 g BEPP. In der wässrigen Phase war kein BEPP mehr nachweisbar. Die beiden organischen Phasen wurden vereinigt und das Lösungsmittel zum größten Teil abgezogen. Es verblieben 217,2 g eines Öls mit einem Gehalt von 89,1 Gew.-% BEPP (HPLC, externer Standard) zurück. Das entspricht einer Ausbeute einer 89,5 % der Theorie.

### Beispiel 3

In einem Kolben wurden unter Stickstoff in einem Gemisch aus 210 g trockenem Tetrahydrofuran und 90 g trockenem Toluol 21,9 g Lithiumaluminiumhydrid vorgelegt. Zu der grauen Suspension wurden bei 90°C 58,8 g 3-Amino-1-benzyl-pyrrolidindion (96,2 %) gelöst in einem Gemisch aus 70 g trockenem Tetrahydrofuran und 30 g trockenem Toluol aus einem Tropftrichter eindosiert. Die anfangs mäßige H₂-Entwicklung wurde mit der Zeit lebhafter. Die Rückflußtemperatur fiel kontinuierlich auf 76°C ab. Nach beendeter Zugabe wurde noch 5 Stunden bei Rückfluß nachgerührt und die Suspension auf 20°C abgekühlt. Die dünnflüssige Suspension wurde in einen trockenen, mit Stickstoff gespülten Tropftrichter überführt. In einem zweiten Kolben wurden 145,2 g Citronensäure (Monohydrat) und 33,9 g Schwefelsäure (100 %ig) in 600,0 g Wasser vorgelegt. Zu dieser Lösung dosierte man aus dem Tropftrichter die Reaktionssuspension in 1 Stunde bei 10°C (H₂-Entwicklung). Es bildete sich eine organische und eine wässrige Phase. Es wurde 1 Stunde bei 60°C nachgerührt. Nach Abkühlen des Kolbeninhalts auf 30°C (pH = 2,1) wurde bei dieser Temperatur der pH-Wert durch Zugabe von 360,0 g wässriger Natronlauge (15 %ig) auf einen Wert von 8,5 eingestellt. Danach wurde die Temperatur auf 50°C gesteigert und bei dieser Temperatur 1 Stunde nachgerührt. Die organische Phase wurde abgetrennt und die wässrige Phase zweimal mit einem Gemisch aus 140 g Tetrahydrofuran und 60 g Toluol bei 50°C nachextrahiert. Die organischen Phasen wurden vereinigt und das Lösungsmittel zum größten Teil abgezogen. Es hinterblieben 41,5 g eines Öls zurück, das einen Gehalt von 86,7 % (GC) an 3-Amino-1-benzyl-pyrrolidin aufwies (73,4 % d.Th.).

## Patentansprüche

1. Verfahren zur Herstellung von basisch reagierenden, organischen, stickstoffhaltigen Verbindungen durch Reduktion mit Lithiumaluminiumhydrid, **dadurch gekennzeichnet ist, dass** man das nach der Reduktion vorliegende Gemisch hydrolysiert, indem man es zu einer wässrigen Lösung hinzufügt, in der Substanzen gelöst sind, die α-Hydroxycarboxyl- und/oder α-Aminocarboxyl-Gruppen enthalten und die sich dabei bildenden beiden Phasen bei einem pH-Wert der wässrigen Phase von 7 bis 12 trennt.

2. Verfahren nach Anspruch 1, **dadurch gekennzeichnet, dass** man Amine, Pyridine, Piperidinen, Piperazinen, Pyrrole, Pyrrolidinen oder Indole durch Reduktion von Verbindungen herstellt, die neben Amino-, Pyridino-, Piperidino-, Piperazino-, Pyrrolo-, Pyrrolidino- oder Indol-Gruppen eine oder mehrere mit Lithiumaluminiumhydrid reduzierbare Gruppen enthalten.

3. Verfahren nach Anspruch 1, **dadurch gekennzeichnet, dass** man Amine durch Reduktion der entsprechenden Amide, Imide, Imine, Hydrazone, Hydroxylamine, Oxime oder Nitrile herstellt.

4. Verfahren nach Ansprüchen 1 und 2, **dadurch gekennzeichnet, dass** man aus 3-Amino-1-benzyl-pyrrolidindion das 3-Amino-1-benzyl-pyrroline oder aus 8-Benzyl-7,9-dioxo-2,8-diazabicyclo[4.3.0]nonan das 8-Benzyl-2,8-diazabicyclo[4.3.0]-nonan herstellt.

5. Verfahren nach Ansprüchen 1 bis 4, **dadurch gekennzeichnet, dass** als α-Hydroxycarboxylgruppen enthaltende Substanzen α-Hydroxycarbonsäuren mit 1 bis 6 OH-Gruppen und 2 bis 10 C-Atomen eingesetzt werden, wobei sich mindestens eine OH-Gruppe in α-Stellung zu einer Carboxylgruppe befindet und als α-Aminocarboxylgruppen enthaltende Substanzen α-Aminocarbonsäuren mit 1 bis 4 Aminogruppen und 2 bis 20 C-Atomen, wobei sich mindestens eine primäre, sekundäre oder tertiäre Aminogruppe in α-Stellung zu einer Carboxylgruppe befindet.

6. Verfahren nach Ansprüchen 1 bis 5, **dadurch gekennzeichnet, dass** man 1 bis 3 Äquivalente α-Hydroxycarboxylgruppen und α-Aminocarboxylgruppen enthaltende Substanzen, bezogen auf 1 Mol eingesetztes Lithiumaluminiumhydrid einsetzt.

7. Verfahren nach Ansprüchen 1 bis 6, **dadurch gekennzeichnet, dass** die α-Hydroxycarboxylgruppen und α-Aminocarboxylgruppen enthaltenden Substanzen in wässrigen Lösungen mit einer Konzentration von 5 bis 60 Gew.-% eingesetzt werden.

8. Verfahren nach Ansprüchen 1 bis 7, **dadurch gekennzeichnet, dass** man die Phasentrennung bei Temperaturen von 5 bis 100°C durchführt.

9. Verfahren nach Ansprüchen 1 bis 8, **dadurch gekennzeichnet, dass** der pH-Wert der wässrigen Phase vor der Phasentrennung 7,5 bis 9,5 beträgt.

10. Verfahren nach Ansprüchen 1 bis 9, **dadurch gekennzeichnet, dass** man die nach Abtrennung der organischen Phase verbleibende wässrige Phase extrahiert.
